(19) **Europäisches Patentamt**
**European Patent Office**
**Office européen des brevets**

(11) Veröffentlichungsnummer : **0 051 806**
**B1**

(12) # EUROPÄISCHE PATENTSCHRIFT

(45) Veröffentlichungstag der Patentschrift :
28.11.84

(51) Int. Cl.³ : **C 07 C 85/24, C 07 C 87/50**

(21) Anmeldenummer : 81109102.4

(22) Anmeldetag : 28.10.81

(54) Verfahren zur Herstellung von 3,5-Dimethylanilin.

(30) Priorität : 06.11.80 DE 3041899

(43) Veröffentlichungstag der Anmeldung :
19.05.82 Patentblatt 82/20

(45) Bekanntmachung des Hinweises auf die Patenterteilung : 28.11.84 Patentblatt 84/48

(84) Benannte Vertragsstaaten :
CH DE FR GB LI

(56) Entgegenhaltungen :
J. AM. CHEM. SOC., Band 69, August 1947, Seiten 1907-1908 Washington D.C., U.S.A. E.C. HORNING et al.: "Aromatization studies. V. Synthesis of alkylanilines from alkylcyclohexenones"

(73) Patentinhaber : HOECHST AKTIENGESELLSCHAFT
Postfach 80 03 20
D-6230 Frankfurt am Main 80 (DE)

(72) Erfinder : Berthold, Rüdiger, Dr.
Geierfeld 55
D-6232 Bad Soden Taunus (DE)
Erfinder : Müller, Werner Heinrich, Dr.
Taunusblick 5
D-6239 Eppstein/Taunus (DE)

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Aus J. Amer. Chem. Soc. 69 (1947) 1907-1908 ist es bekannt, 3,5-Dimethyl-2-cyclohexen-1-on-azin durch Erhitzen mit einem Palladium-Kohle-Katalysator in einem inerten Lösungsmittel in das 3,5-Dimethylanilin umzuwandeln. Es wird ausgeführt, daß in zahlreichen Experimenten die optimalen Bedingungen für die Aromatisierung des Azins ausgearbeitet wurden, wobei sich als bestes Lösemittel Triethylbenzol herausstellte. Das Dimethylanilin wurde hierbei in einer Ausbeute von 50 % gewonnen. Andere Alkylbenzole ergaben niedrigere Ausbeuten, wie auch der Diphenylether, mit dem maximal 44 % Ausbeute erhalten wurden.

Überraschenderweise wurde nun gefunden, daß wesentlich höhere Ausbeuten bei dieser Reaktion erhalten werden, wenn das Lösemittel ein Ether ist, der mindestens einen aliphatischen Rest enthält.

Die Erfindung betrifft deshalb ein Verfahren zur Herstellung von 3,5-Dimethylanilin aus 3,5-Dimethyl-2-cyclohexen-on-azin durch Erhitzen in einem inerten Lösungsmittel mit einem ein Edelmetall der 8. Nebengruppe des Periodensystems enthaltenden Katalysator, indem man das genannte Azin gelöst in einem Ether, der mindestens einen aliphatischen Rest enthält, bei Temperaturen von 150° bis 350 °C bei Drücken von 0,001 bis 100 bar erhitzt.

Die Herstellung des 3,5-Dimethylanilins aus dem Azin zeichnet sich gegenüber anderen bekannten Verfahren vor allem dadurch aus, daß das Produkt frei von Isomeren erhalten wird. Darüberhinaus ist von Vorteil, daß keine aromatischen Vorprodukte erforderlich sind, sondern von einfachen aliphatischen Bausteinen ausgegangen wird. So ist das 3,5-Dimethyl-2-cyclohexenon aus Acetessigester und Acetaldehyd leicht zugänglich (DE-OS 26 54 850) und daraus mit Hydrazinhydrat in sehr guter Ausbeute das Azin.

Im folgenden werden bevorzugte Ausgestaltungen der Erfindung näher erläutert.

Da es sich bei dem erfindungsgemäßen Verfahren um eine katalytische Dehydrierung im heterogenen System handelt, ist es zweckmäßig, ständig für eine gute Durchmischung des Reaktionssystems zu sorgen. Diese wird durch entsprechendes Rühren oder einfacher durch heftiges Sieden erreicht.

Die Reaktionstemperaturen liegen zwischen 150 und 350 °C, vorzugsweise zwischen 180 und 260 °C, da in diesem Bereich die Reaktion rasch und mit hoher Selektivität abläuft. Je nach Art des eingesetzten Lösemittels wird bei Drücken von 0,001 bis 100, insbesondere von 0,01 bis 20 bar gearbeitet, wobei Druck und Temperatur zweckmäßig so aufeinander abgestimmt sind, daß genügend flüssige Phase vorhanden ist und die erwünschte Reaktionstemperatur aufrechterhalten wird.

Als Lösemittel eignen sich grundsätzlich alle Ether, die mindestens einen aliphatischen Rest enthalten. Vorzugsweise werden Ether mit einem Siedepunkt von mindestens etwa 80 °C eingesetzt, da bei niedriger siedenden Ethern relativ hohe Drücke erforderlich sind, um eine genügend hohe Reaktionstemperatur zu erreichen, bei der die Reaktion in annehmbarer Zeit zu Ende geführt werden kann.

Geeignete höher siedende Mono- oder Dialkylether sind beispielsweise Di-n-butylether, Alkyl-Aryl-Ether, wie Anisol und Phenetol, insbesondere jedoch Polyether, die sich vom Ethylen- und/oder Propylenglykol ableiten. Als solche Polyether kommen im einfachsten Fall die niederen Dialkylether des Ethylenglykols und Propylenglykols in Betracht, vorzugsweise jedoch niedere Dialkylether von Polyglykolen wie Ethylendi-, -trioder -tetraglykol oder von höheren Polyglykolen, weiterhin auch Monoarylether wie Anlagerungsprodukte von Ethylenoxid an Alkylphenole wie Nonylphenol oder Tributylphenol, deren freie Hydroxygruppe mit niederen Alkanolen verethert sein kann. Als Ether kommen jedoch auch höhere Polyglykole mit freien Hydroxy-Endgruppen in Betracht, bevorzugt sind jedoch ihre niederen Dialkylether mit bis zu 6 Kohlenstoffatomen in den Ethergruppen, insbesondere die Methyl- und Ethylether.

Zweckmäßig wählt man einen Ether, der bei atmosphärischem Druck im bevorzugten Temperaturbereich zwischen 180 und 260 °C siedet, da mit diesem Lösemittel drucklos gearbeitet werden kann und die Dehydrierung unter Rückflußbedingungen besonders rasch und selektiv verläuft. Hierbei kann — abhängig von den Reaktionsbedingungen — der Siedepunkt des Lösemittels so gewählt werden, daß sich das 3,5-Dimethylanilin bei der Aufarbeitung im Destillationssumpf oder im Destillat findet. Eine zweckmäßige Ausgestaltung der Erfindung besteht auch darin, daß man einen hochsiedenden Ether einsetzt und das entstandene Amin in dem Maße, wie es entsteht, abdestilliert, gegebenenfalls unter vermindertem Druck. Auf diese Weise kommt man mit einer geringen Menge an Lösemittel aus. Wird ein Ether eingesetzt, der tiefer als das entstehende Amin siedet, so kann man das Lösemittel laufend abdestillieren und — um Lösemittel einzusparen — in den Prozeß zurückführen, wobei das Amin aus dem Sumpf ausgeschleust wird. Diese Verfahren lassen sich auch vollkontinuierlich ausgestalten. Hierbei wird beispielsweise eine Lösung des Azins in demselben Lösemittel, das auch zur Suspendierung des Katalysators verwendet wird, oder das geschmolzene Azin kontinuierlich über einen Vorheizer in den Dehydrierungsreaktor eingebracht, während gleichzeitig, eine entsprechende Menge der Reaktionsmischung, die das gebildete Amin enthält, ausgetragen wird. Der Katalysator wird hierbei beispielsweise mittels einer Fritte im Reaktor zurückgehalten oder nach Abtrennung, z. B. mittels eines Dekanters, in den Reaktor zurückgeführt. Das Lösemittel wird nach destillativer Abtrennung vom gebildeten Amin wieder zum Lösen von neuem Azin verwendet.

Es ist auch möglich, das Azin in einem tiefsiedenden Lösemittel gelöst zuzugeben, das während der Dehydrierung laufend abdestilliert. Als solches tiefsiedendes Lösemittel für das Azin kommen nicht nur Ether, sondern auch andere hinreichend inerte Lösemittel in Betracht, beispielsweise niedere Alkanole wie Isopropanol.

Neben der Art des Lösemittels hat auch die Menge einen gewissen Einfluß auf die Reaktion, da mit steigender Konzentration des eingesetzten Azins und des gebildeten Amins die Ausbeute abnimmt. Grundsätzlich sind die Ausbeuten um so höher, je niedriger die Konzentration ist. Aus wirtschaftlichen Gründen wählt man eine Azin- und Aminkonzentration im Reaktionsgemisch von bis zu etwa 30 Gew.-%, vorzugsweise bis zu etwa 15 %, bezogen auf das Lösemittel.

Um die Azinkonzentration möglichst niedrig zu halten, stellt man zweckmäßig die Geschwindigkeit der Azinzugabe auf die Dehydrier-Kapazität des eingesetzten Katalysators ein, was durch einen einfachen Vorversuch leicht zu ermitteln ist.

Wenn die handelsüblichen Trägerkatalysatoren eingesetzt werden, bei denen das Edelmetall auf einen inerten, oberflächenreichen Träger aufgebracht ist, wählt man eine Katalysator-Teilchengröße von etwa 0,01 bis 5 mm, vorzugsweise 0,05 bis 1 mm. Abhängig vom Lösemittel und vom Katalysator kann die Reaktions-Suspension 0,1 bis 40 % Trägerkatalysator, bezogen auf das Gewicht des flüssigen Reaktionsmediums, enthalten. Bevorzugt sind 1 bis 30 %, bezogen auf das Gewicht des Lösemittels.

Die Aktivität des Katalysators nimmt im Laufe der Reaktion allmählich ab, wobei im gleichen Maße Nebenprodukte (Di- und Triarylamine, Carbazole) gebildet werden. Diese Nebenprodukte bleiben bei der destillativen Abtrennung des Amins im Rückstand. Zur Abtrennung vom Lösemittel kann der vom Katalysator abgesaugte Destillationsrückstand auf Wasser gegossen werden, worauf die ungelösten sekundären und tertiären Amine abgetrennt und das mit Kohle geklärte wäßrige Filtrat durch Destillation entwässert wird.

In den folgenden Beispielen beziehen sich Prozentangaben auf das Gewicht, sofern nichts anderes angegeben ist.

Beispiele

1. 12,5 g 3,5-Dimethyl-2-cyclohexenon-azin, 98 %ig (entsprechend 12,2 g 100 %iges Produkt = 0,05 Mol) werden in 100 ml Isopropanol gelöst. Diese Lösung tropft man innerhalb von 3 Stunden zu einer siedenden Suspension (Kp. 215 °C) von 10 g Palladium-Trägerkatalysator (UM 45 der Firma Universal-Matthey Products) in 100 g Methylbutyldiglykol. Über eine kleine Raschigkolonne destillieren 100 ml Isopropanol ab. Wenn die Azinlösung zugetropft ist, rührt man noch 15 Minuten bei Siedetemperatur nach, kühlt auf Raumtemperatur und saugt vom Katalysator ab.

Man erhält 88 g Lösung.

Bei der gaschromatographischen Analyse findet man in dieser Lösung 12,7 % 3,5-Dimethylanilin entsprechend 11,2 g (= 0,092 4 Mol) oder 92,4 % d. Th.

2. Die Umsetzung erfolgt wie in Beispiel 1, die isopropanolische Azinlösung wird jedoch innerhalb von 6 Stunden zugetropft.

Man erhält 92 g Lösung mit einem Gehalt von 12,5 %, entsprechend 11,5 g 3,5-Dimethylanilin (= 0,095 Mol) oder 95 % d. Th.

3. Der Dimethylether eines Polyethylenglykols vom mittleren Molgewicht 200 (Siedebereich 240-350 °C) wird an einer Kolonne bei 0,4 mbar bis Kp. 225 °C fraktioniert. Der Destillationsrückstand wird mit Kohle geklärt. Zu 150 g dieses Rückstandes gibt man 10 g Palladiumträgerkatalysator (UM 45), heizt bei einem Vakuum von ca. 26 mbar auf ca. 210 °C und tropft mit einer Geschwindigkeit von ca. 1 g pro Minute unter guter Rührung geschmolzenes, rohes 3,5-Dimethyl-2-cyclohexenonazin (Reingehalt 98 %, Fp. 71-79 °C) zu. Es entsteht 3,5-Dimethylanilin, das zusammen mit etwas zersetztem Polyethylenglykol-dimethylether überdestilliert. Nach etwa 8 Stunden sind 500 g 3,5-Dimethyl-2-cyclohexenonazin zugetropft. Man erhält nach Zugabe der ersten 250 g Azin (= 1,0 Mol) 249 g Destillat, enthaltend 92 %, also 229 g 3,5-Dimethylanilin (= 1,89 Mol), entsprechend 94,6 % d. Th.

Nach Zugabe der 2. Hälfte Azin erhält man 236 g Destillat mit einem Amingehalt von 89,6 %, entsprechend 211 g 3,5-Dimethylanilin (= 1,75 Mol) oder 87 % d. Th.

Die Gesamtausbeute beträgt 440 g 3,5-Dimethylanilin, das sind 90,9 % d. Th.

4. In einem 1,5 l Edelstahlreaktor, ausgerüstet mit Rührer, Rückflußkühler und automatischer Stand- und Druckhaltung werden 50 g eines 2,8 % $Pd/Al_2O_3$-Pulver-Katalysators und 1 l Dimethyldiglykol gegeben. Unter $N_2$-Spülung wird aufgeheizt. Die automatische Druckhaltung wird auf 1,6 bar eingestellt, wobei sich eine Rückflußtemperatur von 190 °C einstellt. Nun werden stündlich 100 g 3,5-Dimethyl-2-cyclohexenonazin, gelöst in 900 g Dimethyldiglykol, zugepumpt und gleichzeitig aus dem Reaktor 1 000 g Reaktionsprodukt über eine Filtervorrichtung, die den Katalysator im Reaktor zurückhält, abgenommen. Der entstehende Wasserstoff wird über die automatische Druckhaltung abgelassen, wenn der Reaktordruck 1,6 bar übersteigt.

Die gaschromatographische Analyse des Reaktionsprodukts zeigt 8,9 % 3,5-Dimethylanilin. Nach Abdestillieren des Dimethyldiglykols destilliert bei 93 °C/12 mbar 99,6 %iges 3,5-Dimethylanilin über.

**Ansprüche**

1. Verfahren zur Herstellung von 3,5-Dimethylanilin aus 3,5-Dimethyl-2-cyclohexen-on-azin durch Erhitzen in einem inerten Lösemittel mit einem ein Edelmetall der 8. Nebengruppe des Periodensystems enthaltenden Katalysator, dadurch gekennzeichnet, daß man das genannte Azin gelöst in einem Ether, der mindestens einen aliphatischen Rest enthält, bei Temperaturen von 150° bis 350 °C bei Drücken von 0,001 bis 100 bar erhitzt.

2. Verfahren nach Anspruch 1, dadurch gekennzeichnet, daß der Ether ein niederer Dialkylether eines Polyethylenglykols ist.

3. Verfahren nach Anspruch 1 oder 2, dadurch gekennzeichnet, daß die Konzentration des Azins im Reaktionsgemisch 30 Gewichtsprozent nicht überschreitet.

4. Verfahren nach Ansprüchen 1-3, dadurch gekennzeichnet, daß die Konzentration des Azins 15 Gewichtsprozent, bezogen auf das Lösemittel, nicht überschreitet.

5. Verfahren nach Ansprüchen 1-4, dadurch gekennzeichnet, daß man das Azin zum erhitzten Lösemittel zugibt.

## Claims

1. A process for the preparation of 3,5-dimethylaniline from 3,5-dimethyl-2-cyclohexenone azine by heating it in an inert solvent with a catalyst containing a noble metal of the 8th auxiliary group of the Periodic Table of Elements, which comprises heating the said azine, dissolved in an ether containing at least an aliphatic radical, at a temperature of from 150° to 350 °C at a pressure of from 0,001 to 100 bar.

2. The process as claimed in claim 1, wherein the ether is a low molecular dialkylether of polyethyleneglycol.

3. The process as claimed in claim 1 or 2, wherein the concentration of the azine in the reaction mixture does not pass over 30 per cent by weight.

4. The process as claimed in claims 1-3, wherein the concentration of the azine does not pass over 15 per cent by weight, referred to the solvent.

5. The process as claimed in claims 1-4, wherein the azine is added to the heated solvent.

## Revendications

1. Procédé pour préparer la diméthyl-3,5 aniline à partir de la diméthyl-3,5 cyclohexène-2 one-azine par chauffage dans un solvant inerte en présence d'un catalyseur contenant un métal noble du huitième sous-groupe de la Classification Périodique, procédé caractérisé en ce qu'on chauffe l'azine mentionnée, sous la forme d'une solution dans un éther contenant au moins un radical aliphatique, à des températures de 150 à 350 °C et sous des pressions de 0,001 à 100 bar.

2. Procédé selon la revendication 1 caractérisé en ce que l'éther est un éther dialkylique inférieur d'un polyéthylène-glycol.

3. Procédé selon l'une des revendications 1 ou 2, caractérisé en ce que la concentration du mélange réactionnel en l'azine ne dépasse pas 30 % en poids.

4. Procédé selon l'une quelconque des revendications 1 à 3, caractérisé en ce que la concentration en l'azine ne dépasse pas 15 % en poids par rapport au solvant.

5. Procédé selon l'une quelconque des revendications 1 à 4, caractérisé en ce qu'on ajoute l'azine au solvant chauffé.